# EUROPEAN PATENT APPLICATION

(11) **EP 4 609 704 A1**
(43) Date of publication of application: **03.09.2025**
(21) Application number: 23882667.1
(22) Date of filing: 25.10.2023
(51) Int. Cl.: A01H 6/08

(54) **DOUBLE-FLOWERED MANDEVILLA GENUS PLANT AND METHOD FOR BREEDING SAME**

(30) Priority: 28.10.2022 JP 2022173593
(71) Applicant: Suntory Flowers Limited, Tokyo 108-0014 (JP)
(72) Inventor: MISATO, Tomoya, Tokyo 108-0014 (JP)
(74) Representative: Mewburn Ellis LLP
(86) International application number: PCT/JP2023/038455
(87) International publication number: WO 2024/090462

(57) **Abstract**

It is possible to create Mandevilla plants, and especially double blooming Mandevilla plants, which have not been hitherto created. Provided is the Mandevilla plant deposited as Accession No. FERM BP-22460 at the independent administrative International Patent Organism Depositary of the National Institute of Technology and Evaluation (NITE-IPOD), and its progeny.

## Description

### FIELD

The present invention relates to a double blooming Mandevilla plant and to a method for creating it.

### BACKGROUND

Plants of the genus Mandevilla (also known as Dipladenia) are climbing woody or upright herbaceous plants belonging to the Apocynaceae family, with about 130 native species found across the tropical Americas from Mexico to Argentina.

Mandevilla plants are very popular as garden plants because they produce beautiful flowers for long periods, and in recent years many different varieties have been created by cross-breeding. Varieties with double blooming flowers have high ornamental value due to a beautiful appearance, and they tend to be preferred for horticulturing.

The known double blooming Mandevilla varieties are branches of the Alice du Pont line, such as Rita Marie Green which has dark pink flowers (PTL 1), but double blooming varieties of Mandevilla are still extremely rare, and creation of new varieties is therefore desired.

### [CITATION LIST]

### [PATENT LITERATURE]

[PTL 1] U.S. Plant Patent No. 6300547

### SUMMARY

### [TECHNICAL PROBLEM]

Mandevilla plants have special flower structures and are technologically difficult to artificially cross-breed by artificial pollination. Although about 130 native species are known to exist, only 10 of these are used in horticulture. As mentioned above, "Rita Marie Green" is known as an existing double bloom Mandevilla cultivar, but no double blooming individuals have yet been obtained out of about 100 F1 individuals obtained by hybridization with the cultivar, about 500 seedlings of the B1 generation obtained by backcrossing, and about 500 seedlings of the second B2 generation obtained by continuous backcrossing.

Conventional "Rita Marie Green" has disadvantages in that it is usually distributed in large containers, being difficult to produce in small flowering pots, in that it grows as a vigorous vine to a large size, in that it is unsuitable for small containers (being mainly distributed in 4-inch pots in Europe and America), in that it has a low appreciation level due to large unevenness between the leaf veins and hairiness and poor texture of the leaves, and in that long times are required for flowering after cuttings have been taken.

It is an object of the invention to provide a novel double blooming Mandevilla plant and a method for creating it.

### [SOLUTION TO PROBLEM]

The present inventors have repeatedly carried out a total of more than 14,000 cutting propagations of "Cream Pink" (Suntory Flowers) as a single blooming Mandevilla cultivar, over a period of 16 years since initial marketing of "Sun Parasol^{™} (solid under the name of Milky Pink"), and have succeeded in creating from among the cultivars a "Double Pink" cultivar with pink flower color, as the sole mutant with double blooming flowers. A cultured seedling of "Double Pink" was deposited internationally at the International Patent Organism Depositary (IPOD) of the independent administrative National Institute of Technology and Evaluation (NITE-IPOD) (Room 120, 2-5-8 Kazusa Kamatari, Kisarazu City, Chiba Prefecture) on October 14, 2022, in accordance with the Budapest Treaty (Designation: Sunpa421: Accession No.: FERM BP-22460).

Even more surprisingly, after repeatedly collecting approximately 3000 cuttings from "Double Pink", a branch was discovered having double blooming flowers with a deep red flower color, and the double bloom cultivar "Double Red" was successfully created.

Specifically, the present invention provides the following.
[1] The Mandevilla plant deposited as Accession No. FERM BP-22460 at the independent administrative International Patent Organism Depositary of the National Institute of Technology and Evaluation (NITE-IPOD), or its progeny.
[2] A Mandevilla plant created by vegetative propagation of the Mandevilla plant according to [1] above.
[3] A Mandevilla plant according to [2] above, wherein the vegetative propagation is carried out with cuttings using branches with at least one double blooming flower.
[4] A Mandevilla plant according to [3] above, wherein the branches with at least one double blooming flower are bud sports.
[5] A breeding material, a partial plant body, a tissue or cells of a Mandevilla plant according to any one of [1] to [4] above.
[6] A method for creating a Mandevilla plant, the method including a step of providing a Mandevilla plant selected from among the Mandevilla plant deposited as Accession No. FERM BP-22460 at the independent administrative International Patent Organism Depositary of the National Institute of Technology and Evaluation (NITE-IPOD), and its progeny.
[7] The method according to [6] above, which further includes a step of vegetative propagation of the Mandevilla plant deposited as Accession No. FERM BP-22460 at the International Patent Organism Depositary (NITE-IPOD), or its progeny.
[8] The method according to [7] above, wherein the vegetative propagation is carried out with cuttings using branches with at least one double blooming flower.
[9] The method according to [8] above, wherein the branches with at least one double blooming flower are bud sports.
[10] The method according to any one of [6] to [9] above, wherein the Mandevilla plant created by the method is a double blooming Mandevilla plant.

### [ADVANTAGEOUS EFFECTS OF INVENTION]

According to the invention it is possible to create novel double blooming Mandevilla plants having excellent characteristics compared to existing double blooming Mandevilla plants. The following is a comparison between "Rita Marie Green", as an existing double blooming Mandevilla plant cultivar, and "Double Pink", as a double blooming Mandevilla plant cultivar of the invention.
· "Rita Marie Green" (scientific name: *Mandevilla x amabilis*) and "Double Pink" (scientific name: *Mandevilla sanderi*) are clearly distinguished as different and separate species.
· "Rita Marie Green" is distributed mainly in large containers and is difficult to produce in small flowering pots, whereas the "Double Pink" cultivar can be produced and distributed in 4-inch flowering pots that are common in Europe and America.
· "Rita Marie Green" grows as a vigorous vine of a large size which is unsuitable for distribution in small containers (4-inch pots commonly used in Europe and America), whereas the cultivar "Double Pink" grows upright with no vines or virtually no vines, and is therefore suitable for distribution in small containers (4-inch pots common in Europe and America).
· The middle part of "Rita Marie Green" has an internodal length of 15 to 20 cm on average, making it difficult to cultivate in small containers, whereas that of the cultivar "Double Pink" is as narrow as 2 cm making it suitable for 4-inch cultivation, thus improving the productivity of seedling cuttings produced from a single parent stock.
· "Rita Marie Green" is highly uneven between the leaf veins and has hairiness and a rough leaf texture, giving it a low degree of appreciation, whereas the cultivar "Double Pink" has no unevenness, instead having a smooth, glossy surface texture for the leaves.
· The flowering period for "Rita Marie Green" is 30 weeks as a seedling, assuming cutting at 44 weeks, whereas the same for the cultivar "Double Pink" is more than 6 weeks earlier at 24 weeks, and therefore productivity is higher.

Thus, the double blooming Mandevilla plant cultivar of the invention has very properties that are superior to the existing cultivar.

### BRIEF DESCRIPTION OF DRAWINGS

Fig. 1 is a photograph of "Double Pink", a double blooming Mandevilla plant cultivar of the present invention.
Fig. 2 is a photograph of "Double Red", a double blooming Mandevilla plant cultivar of the present invention.

### DESCRIPTION OF EMBODIMENTS

One aspect of the invention provides a double blooming Mandevilla plant, the double blooming Mandevilla plant being the Mandevilla plant deposited as Accession No. FERM BP-22460 at the independent administrative International Patent Organism Depositary of the National Institute of Technology and Evaluation (NITE-IPOD), or its progeny.

As mentioned above, the pink flower color cultivar "Double Pink" was created as the only double blooming mutant from "Cream Pink", an existing single blooming Mandevilla cultivar. The cultured seedlings were deposited internationally at the International Patent Organism Depositary (IPOD) of the independent administrative National Institute of Technology and Evaluation (NITE-IPOD) (Room 120, 2-5-8 Kazusa Kamatari, Kisarazu City, Chiba Prefecture) on October 14, 2022, in accordance with the Budapest Treaty (Designation: Sunpa421: Accession No.: FERM BP-22460).

The term "double blooming" refers to a type of flowering in which the petals overlap. With double blooming flowers, more petals align where the stamen or pistils are normally aligned inside the petals of single blooming flowers, making it appear that the flower is composed of petals alone. Since wild plants usually have stamens and pistils, double blooming flowers generally appear by mutation. The inner petals of double blooming flowers correspond to the stamens or pistils, but have petalized to form double blooming flowers. As used herein, "double blooming" refers to this property of flowers, and it is sufficient if the double blooming Mandevilla plant of the invention has at least one double blooming flower. A double blooming flower typically comprises a total of 10 petals, including five petals formed by stamen petalization in addition to the five petals normally present in single blooming flowers.

As used herein, the term "progeny" includes self-pollinated or outbred progeny of the Mandevilla plant deposited as Accession No. FERM BP-22460 at the independent administrative International Patent Organism Depositary of the National Institute of Technology and Evaluation (NITE-IPOD), and not only first generation but also all generation descendants of the Mandevilla plant deposited as Accession No. FERM BP-22460 at the independent administrative International Patent Organism Depositary of the National Institute of Technology and Evaluation (NITE-IPOD). Moreover, the term "progeny" as used herein includes plants created from parent Mandevilla plants based on conventional technologies for cross-breeding of plants, such as conventional breeding techniques including vegetative propagation or seed breeding of parent plants, as well as tissue culture techniques for culturing of plant cells, tissues or organs, and may also include those created by gene recombinant technology which allows direct introduction of useful traits, and mutants such as bud sports of parent Mandevilla plants. Such progeny preferably have the desired properties of the parent Mandevilla plant (especially the double blooming property).

Mandevilla plants of the invention can be created using conventional technology for cross-breeding of plants from the Mandevilla plant deposited as Accession No. FERM BP-22460 at the independent administrative International Patent Organism Depositary of the National Institute of Technology and Evaluation (NITE-IPOD) (i.e. "Double Pink"), or its progeny. Such conventional technology may include conventional breeding techniques such as vegetative propagation and seed breeding, as well as tissue culture techniques for culturing of plant cells, tissues or organs, and gene recombinant technology which allows direct introduction of useful traits. Mandevilla plants of the invention can typically be created by vegetative propagation the Mandevilla plant deposited as Accession No. FERM BP-22460 at the independent administrative International Patent Organism Depositary of the National Institute of Technology and Evaluation (NITE-IPOD) (i.e. "Double Pink"), or its progeny. The "vegetative propagation" may be, for example, a method such as cutting (including leaf cutting), layering, stooling (mound layering), division and grafting, or clone seedlings produced by tissue culture may also be used, but cutting is preferred.

Here, "cutting" refers to an asexual reproduction method in which a part of the plant that does not have both stems or roots is cut, and independent individual plants having both stems and roots are grown from them, and the cutting may be branch cutting, stem cutting, root cutting or leaf cutting, depending on the part used. Cutting is typically soil planting of the branches or stems of the parent plant, with a portion exposed on the ground surface. According to the invention, the parts to be used for "cutting" are not particularly restricted but are preferably branches with at least one double blooming flower.

Cuttings may be bud sports from the Mandevilla plant deposited as Accession No. FERM BP-22460 at the independent administrative International Patent Organism Depositary of the National Institute of Technology and Evaluation (NITE-IPOD) (i.e. "Double Pink"), or its progeny. The term "bud sport" refers to a phenomenon in which the flowers, sprouts or leaves of only one particular branch of a plant have mutated at a growth point to have a different genetic trait than the individual, or to an individual expressing such a phenomenon. In plants the body grows outward from the growth point toward the tips, and these parts are therefore distinct from the non-mutated parts, allowing traits to be fixed. Cutting using a sport branch with at least one double blooming flower can therefore create novel cultivars of Mandevilla plants with other properties while maintaining the desired properties (especially the double blooming property). The present inventors actually discovered, surprisingly, a branch with a double blooming deep red flower color during repeated collection of about 3000 cuttings from the Mandevilla plant deposited as Accession No. FERM BP-22460 at the independent administrative International Patent Organism Depositary of the National Institute of Technology and Evaluation (NITE-IPOD) ("Double Pink"), and by propagating it by cutting, have succeeded in creating "Double Red" as a novel double blooming cultivar having a different flower color than Double Pink.

The invention also provides, according to a different aspect, breeding materials (propagules such as cuttings, or seeds), partial plant bodies (such as flowers, stems, branches, leaves or roots), tissues or cells of a Mandevilla plant or its progeny.

According to yet another aspect, the invention also provides a product derived from a Mandevilla plant or its progeny, and typically a potted plant, cut flower or a processed plant created from the cut flower.

According to yet another aspect, the invention provides a method for creating a Mandevilla plant, the method including a step of providing a Mandevilla plant selected from among the Mandevilla plant deposited as Accession No. FERM BP-22460 at the independent administrative International Patent Organism Depositary of the National Institute of Technology and Evaluation (NITE-IPOD), and its progeny.

A person skilled in the art can create a novel Mandevilla plant based on conventional technology for plant cross-breeding, using the Mandevilla plant deposited as Accession No. FERM BP-22460 at the independent administrative International Patent Organism Depositary of the National Institute of Technology and Evaluation (NITE-IPOD), or its progeny, as the provided Mandevilla plant. Such conventional technology may include conventional breeding techniques such as vegetative propagation and seed breeding, as well as tissue culture techniques for culturing of plant cells, tissues or organs, and gene recombinant technology which allows direct introduction of useful traits.

The method of the invention will typically be carried out by vegetative propagation of the Mandevilla plant deposited as Accession No. FERM BP-22460 at the International Patent Organism Depositary (NITE-IPOD), or its progeny, and is preferably carried out with cuttings using a branch with at least one double blooming flower.

By using the method of the invention it is possible to create a Mandevilla plant having desired properties (especially a double blooming property), which could not be created in the past.

### EXAMPLES

### Example 1. Creation of double blooming Mandevilla cultivar "Double Pink"

Cultivation of the single blooming parent Mandevilla cultivar "Cream Pink" with a purple pink (RHS Color Chart: 62C) flower color, as a producing parent variety, was begun in June, 2019 in a greenhouse of VOF Koene & ZN Co. in Maasland, Netherlands. The greenhouse temperature was regulated to a minimum of 18°C, and cultivation was in 20 cm pots. A double blooming individual having a pink (RHS Color Chart: 65C) flower color was obtained from among the cultivated parent varieties in September, 2020. Branches of the individual were cut and raised and managed in 20 cm pots at a minimum temperature of 18°C in a greenhouse at Moerheim New Plant B.V. Co. in Leimuiderbrug, Netherlands. Cutting was repeated to confirm that double blooming flowers were stably obtained.

Cuttings were obtained from the line obtained at Moerheim New Plant B.V. in April, 2021, and imported to the Global Breeding & Resource Center at Suntory Flowers (Higashiomi City). In a greenhouse of the Global Breeding & Resource Center at Suntory Flowers, the cuttings were raised and managed in 9 cm pots at a minimum temperature of 16°C, and cuttings were again obtained in October, 2021. After raising in 9 cm pots in January, 2022, and raising seedlings in a greenhouse regulated at the same minimum temperature of 16°C, they were raised in 18 cm pots and used for a cultivation test in an outdoor field of the same company in May, 2022. The stability of double blooming and flower color were confirmed until August 31, 2022, at which time the test was completed. The double blooming Mandevilla cultivar obtained by the test was designated as "Double Pink".

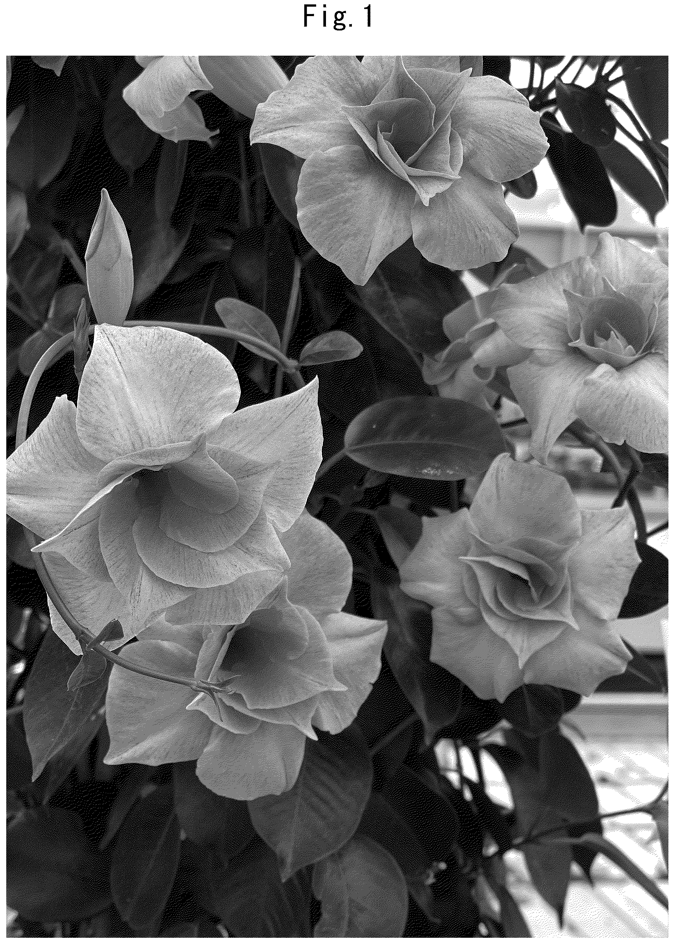

Fig. 1 shows a photograph of a "Double Pink" flower.

The properties of "Double Pink" are as follows.
· The cultivar grows upright with no vines, or is very unlikely to have vines.
· The internodal length at the middle part is narrow, with an average of 2 cm, and no hairs on the stems.
· The overall leaf form is elliptical, with acute leaf tips and round leaf bases.
· Leaf attachment throughout the plant is dense, with an average leaf size of 7.6 cm × 5.4 cm.
· There is no unevenness between the leaf veins, providing a smooth texture on the leaf surface. No hair is found on either the fronts or backs of the leaves.
· The flowering period is at 24 weeks as a seedling, if cutting is at 44 weeks.
· Approximately 3 flowers bloom per branch.
· The corolla diameter is about 6.5 cm.
· The five original petals and the petals forming the double bloom by petalization of the stamen constitute a total of 10 petals.
· Only pistils are present as reproductive organs.
· The petals have a pink (RHS Color Chart: 65C) flower color.
· The petal texture is smooth.
· The corolla tube length is about 3.1 cm and the flower tube is about 1.3 cm.

A cultured seedling of "Double Pink" was deposited internationally at the International Patent Organism Depositary (IPOD) of the independent administrative National Institute of Technology and Evaluation (NITE-IPOD) (Room 120, 2-5-8 Kazusa Kamatari, Kisarazu City, Chiba Prefecture) on October 14, 2022, in accordance with the Budapest Treaty (Accession No.: FERM BP-22460).

### Example 2. Creation of double blooming Mandevilla cultivar "Double Red"

The "Double Pink" cultivar that completed growth as a producing parent in the greenhouse at VOF Koene & ZN in Maasland, Netherlands was cultivated while confirming stability. The greenhouse temperature was regulated to a minimum of 18°C, and cultivation was in 20 cm pots. During repeated collection of approximately 3000 cuttings, one branch having double blooming flowers with a deep red (RHS Color Chart: 53A) flower color was discovered in May, 2022. The branch was raised and managed in 20 cm pots at the same company and in a greenhouse (minimum temperature: 18°C) at Moerheim New Plant B.V. Co. in Leimuiderbrug, Netherlands. Cutting was repeated to confirm that double blooming flowers were stably obtained. The double blooming Mandevilla cultivar obtained by the test was designated as "Double Red".

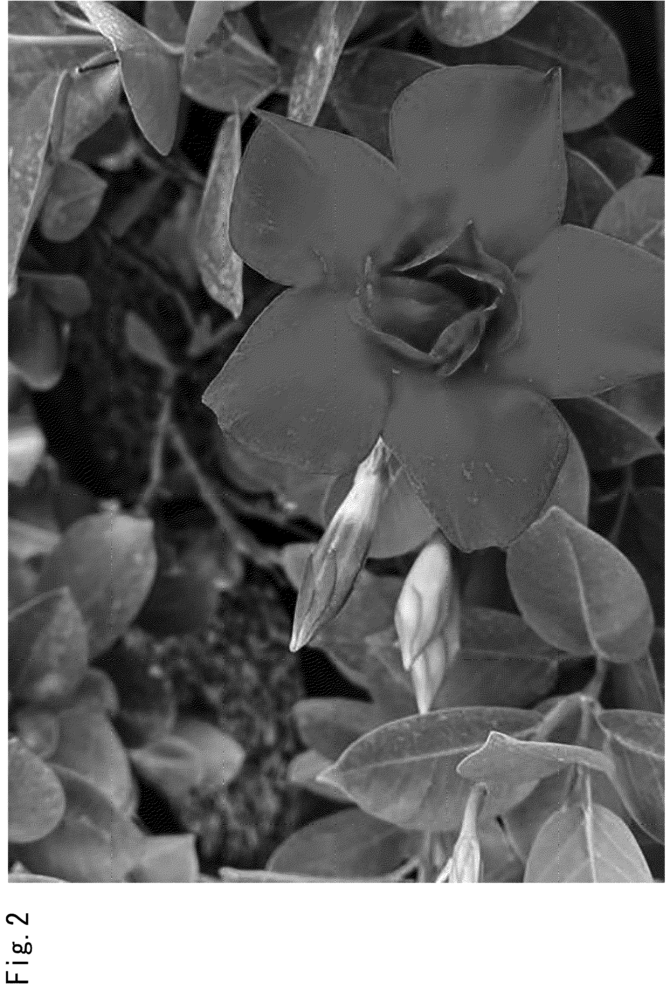

Fig. 2 shows a photograph of a "Double Red" flower.

The properties of "Double Red" are as follows.
· The cultivar grows upright with no vines, or is very unlikely to have vines.
· The internodal length at the middle part is narrow, with an average of 2 cm, and no hairs on the stems.
· The overall leaf form is elliptical, with acute leaf tips and round leaf bases.
· Leaf attachment throughout the plant is dense, with an average leaf size of 7.6 cm × 5.4 cm.
· There is no unevenness between the leaf veins, providing a smooth texture on the leaf surface. No hair is found on either the fronts or backs of the leaves.
· The flowering period is at 24 weeks as a seedling, if cutting is at 44 weeks.
· Approximately 3 flowers bloom per branch.
· The corolla diameter is about 6.5 cm.
· The five original petals and the petals forming the double bloom by petalization of the stamen constitute a total of 10 petals.
· Only pistils are present as reproductive organs.
· The petals have a deep red (RHS Color Chart: 53A) flower color.
· The petal texture is smooth.
· The corolla tube length is about 3.1 cm and the flower tube is about 1.3 cm.

## Claims

1. The Mandevilla plant deposited as Accession No. FERM BP-22460 at the independent administrative International Patent Organism Depositary of the National Institute of Technology and Evaluation (NITE-IPOD), or its progeny.

2. A Mandevilla plant created by vegetative propagation of the Mandevilla plant according to claim 1.

3. A Mandevilla plant according to claim 2, wherein the vegetative propagation is carried out with cuttings using branches with at least one double blooming flower.

4. A Mandevilla plant according to claim 3, wherein the branches with at least one double blooming flower are bud sports.

5. A breeding material, a partial plant body, a tissue or cells of a Mandevilla plant according to any one of claims 1 to 4.

6. A method for creating a Mandevilla plant, the method including a step of providing a Mandevilla plant selected from among the Mandevilla plant deposited as Accession No. FERM BP-22460 at the independent administrative International Patent Organism Depositary of the National Institute of Technology and Evaluation (NITE-IPOD), and its progeny.

7. The method according to claim 6, which further includes a step of vegetative propagation of the Mandevilla plant deposited as Accession No. FERM BP-22460 at the International Patent Organism Depositary (NITE-IPOD), or its progeny.

8. The method according to claim 7, wherein the vegetative propagation is carried out with cuttings using branches with at least one double blooming flower.

9. The method according to claim 8, wherein the branches with at least one double blooming flower are bud sports.

10. The method according to any one of claims 6 to 9, wherein the Mandevilla plant created by the method is a double blooming Mandevilla plant.
